(19) Europäisches Patentamt
European Patent Office
Office européen des brevets

(11) Veröffentlichungsnummer: **0 155 335**
**A1**

(12) # EUROPÄISCHE PATENTANMELDUNG

(21) Anmeldenummer: **84103119.8**

(22) Anmeldetag: **21.03.84**

(51) Int. Cl.$^4$: **C 07 C 45/71**
C 07 C 45/70, C 07 C 45/63
C 07 C 47/575, C 07 C 47/56-5

(43) Veröffentlichungstag der Anmeldung:
**25.09.85 Patentblatt 85/39**

(84) Benannte Vertragsstaaten:
**AT BE CH DE FR GB IT LI LU NL SE**

(71) Anmelder: **LUDWIG HEUMANN & CO GMBH**
**Heideloffstrasse 18-28 Postfach 2260**
**D-8500 Nürnberg(DE)**

(72) Erfinder: **Ahrens, Kurt Henning, Dr.**
**Praterstrasse 9**
**D-8500 Nürnberg(DE)**

(72) Erfinder: **Liebenow, Walter, Dr.**
**Ganghoferstrasse 23**
**D-8500 Nürnberg(DE)**

(72) Erfinder: **Grafe, Ingomar, Dr.**
**Auerbachstrasse 16**
**D-8500 Nürnberg(DE)**

(74) Vertreter: **Kraus, Walter, Dr. et al,**
**Patentanwälte Kraus Weisert & Partner**
**Thomas-Wimmer-Ring 15**
**D-8000 München 22(DE)**

(54) **Verfahren zur Herstellung von 3,5-Dimethoxy-4-alkoxybenzaldehyden.**

(57) Es wird ein Verfahren zur Herstellung von 3,5-Dimethoxy-4-alkoxybenzaldehyden und insbesondere von 3,4,5-Trimethoxybenzaldehyd, ausgehend von einem 4-Hydroxybenzaldehyd der Formel II

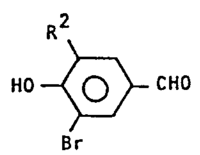

(II)

in der $R^1$ für ein Wasserstoffatom oder eine Methoxygruppe steht, beschrieben. Das erfindungsgemäße Verfahren liefert den angestrebten Aldehyd in sehr guter Ausbeute und ohne das Auftreten von störenden Nebenreaktionen.

EP 0 155 335 A1

KRAUS · WEISERT & PARTNER 0215335

PATENTANWÄLTE

UND ZUGELASSENE VERTRETER VOR DEM EUROPÄISCHEN PATENTAMT

DR. WALTER KRAUS DIPLOMCHEMIKER · DR.-ING. DIPL.-ING. ANNEKÄTE WEISERT · DIPL.-PHYS. JOHANNES SPIES

IRMGARDSTRASSE 15 · D-8000 MÜNCHEN 71 · TELEFON 089/797077

TELEGRAMM KRAUSPATENT · TELEX 5-212156 kpat d · TELEFAX (089) 7918233

4378 WK/an

VERFAHREN ZUR HERSTELLUNG VON 3,5-DIMETHOXY-4-ALKOXYBENZALDEHYDEN



## BESCHREIBUNG

Die Erfindung betrifft ein neues vereinfachtes Verfahren zur Herstellung von 3,5-Dimethoxy-4-alkoxybenzaldehyden der allgemeinen Formel I

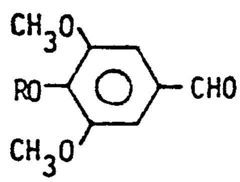

(I)

in der R für eine Niedrigalkyl- oder Niedrigalkoxyethylengruppe, insbesondere eine Methyl- oder Methoxyethylengruppe, steht, aus dem entsprechenden 4-Hydroxybenzaldehyd. Unter "Niedrigalkyl" bzw. "Niedrigalkoxy" wird hierin eine Gruppe mit 1 bis 6 Kohlenstoffatomen, vorzugsweise 1 bis 3 Kohlenstoffatomen, verstanden.

3,5-Dimethoxy-4-alkoxybenzaldehyde, insbesondere 3,4,5-Trimethoxybenzaldehyde, stellen wichtige Zwischenprodukte zur Herstellung von Pharmachemikalien, wie zum Beispiel Trimethoprim, dar. Zur Herstellung dieser Aldehyde sind bereits Verfahren bekannt, bei denen Vanillin oder 4-Hydroxybenzaldehyd bromiert wird und das Bromatom mit Natriummethylat substituiert wird. Entsprechende Verfahren dieser Art werden zum Beispiel in D.V. Rao, F.A. Stuber, Synthesis 1983, 308, der US-A-4 218 567, der DE-PS 723 412 und der DE-OS 26 27 874 beschrieben.

Diese bekannten Verfahren sind aber mit mehreren Nachteilen behaftet. So benötigen sie zum Beispiel relativ große Reaktionsvolumina wegen der geringen Löslichkeit des bromierten 4-Hydroxybenzaldehyds. Weiterhin läuft die Umsetzung nicht vollständig zu Ende ab, so daß das angestrebte Endprodukt nur mit niedrigen Ausbeuten erhalten werden kann. Ein weiterer Nachteil besteht darin, daß diese Verfahren mit Kupfer(I)-Salzen als Katalysator arbeiten, die bei der Umsetzung als metallisches Kupfer ausfallen. Auch treten Nebenreaktionen auf, bei denen eine Methoxygruppe oxidativ als Formalin abgespalten wird, die zu Kondensationsreaktionen unter Harzbildung Anlaß gibt. Schließlich ist die nachträgliche einwandfreie Trennung des bromierten 4-Hydroxybenzaldehyds und Syringaaldehyd äußerst schwierig, und man erhält kein für pharmazeutische Zwecke verwendbares Endprodukt.

Demgegenüber liegt der vorliegenden Erfindung die Aufgabe zugrunde, die oben beschriebenen Nachteile der bekannten Verfahren zu überwinden und den angestrebten 3,5-Dimethoxy-4-alkoxybenzaldehyd mit sehr guter Ausbeute und geeigneter Qualität für die Herstellung der Pharmachemikalien, beispielsweise des Trimethoprims, zu erhalten. Insbesondere soll die Wirtschaftlichkeit bei der Bromierung des 4-Hydroxybenzaldehyds verbessert werden, und beide Bromatome des Brommoleküls sollen in die organische Verbindung eingeführt werden, wodurch Einsparungen hinsichtlich des teuren Broms erhalten werden können. Auch soll der Austausch des aromatisch gebundenen Broms in dem Aldehyd durch die Methoxygruppe ohne Harzbildung bei verringerter Lösungsmittelmenge geschehen, und die zum Endprodukt führende Alkylierung soll auch als Eintopfreaktion durchgeführt werden können.

Diese Aufgabe wird bei einem Verfahren der eingangs erwähnten Art dadurch gelöst, daß man einen 4-Hydroxybenzaldehyd der allgemeinen Formel II

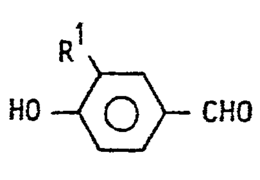

(II)

in der $R^1$ für ein Wasserstoffatom oder eine Methoxygruppe steht, zu einem Aldehyd der allgemeinen Formel III

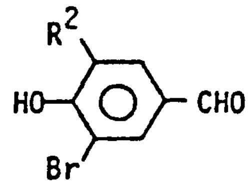

(III)

in der $R^2$ eine Methoxygruppe oder ein Bromatom bedeutet, bromiert, den erhaltenen Aldehyd der Formel II mit Alkalialkoholat in Gegenwart eines Metallkomplexes in Syringaaldehyd der Formel IV

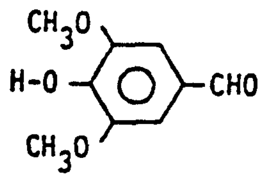

(IV)

überführt und daß man den erhaltenen Syringaaldehyd vorzugsweise ohne Isolierung dieses Zwischenprodukts zu dem gewünschten 3,5-Dimethoxy-4-alkoxybenzaldehyd alkyliert.

In der ersten Stufe des erfindungsgemäßen Verfahrens wird ein 4-Hydroxybenzaldehyd der allgemeinen Formel II

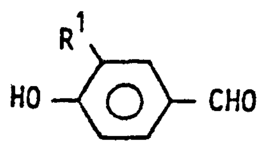

(II)

in der $R^1$ für ein Wasserstoffatom oder eine Methoxygruppe steht, zu einem Aldehyd der allgemeinen Formel III

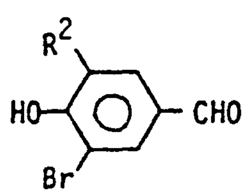

(III)

in der $R^2$ für eine Methoxygruppe oder ein Bromatom steht, bromiert. Die Bromierung des Aldehyds der Formel II erfolgt vorzugsweise in Gegenwart eines Halogenüberträgers,

beispielsweise Succinimid oder N-Bromsuccinimid, oder eines anderen Amids, und in einem Gemisch eines mit Wasser nicht mischbaren Lösungsmittels mit Wasser und einem Alkalibromid, wie Natrium- oder Kaliumbromid. Als mit Wasser nicht mischbares Lösungsmittel wird vorzugsweise ein chlorierter Kohlenwasserstoff, wie Chloroform, Methylenchlorid oder Dichlorethan, verwendet. Die Umsetzungstemperatur liegt im Bereich von 10 bis 80°C, vorzugsweise im Bereich von 30 bis 70°C. Gemäß einer bevorzugten Ausführungsform des erfindungsgemäßen Verfahrens kann die Rückoxidation des entstandenen Bromwasserstoffs mit Chlor durchgeführt werden, wobei überraschenderweise kein Produkt mit aromatisch gebundenem Chlor entsteht. In diesem Falle kann der Aldehyd III mit einer Ausbeute von mehr als 90% erhalten werden, und er zeichnet sich durch hohe Reinheit aus. Das erhaltene Produkt ist in diesem Falle weiß. Führt man die Rückoxidation gemäß einer weniger bevorzugten Ausführungsform des erfindungsgemäßen Verfahrens mit Wasserstoffperoxid durch, dann ist die Ausbeute geringer, und der Aldehyd ist durch Oxidationsprodukte violett gefärbt.

In der zweiten Stufe des erfindungsgemäßen Verfahrens wird das im Aldehyd III vorhandene Brom durch Methoxygruppen substituiert, wodurch man zu dem Syringaaldehyd der Formel IV gelangt. Diese Substitution gelingt dadurch, daß man den Aldehyd III mit Alkalialkoholat in Gegenwart eines Metallkomplexes umsetzt. Als Alkalialkoholat kommen Natriummethylat und Kaliummethylat in Betracht.
Die Verwendung von Natriummethylat in Lösung von Methanol wird bevorzugt. Gemäß einer bevorzugten Ausführungsform des erfindungsgemäßen Verfahrens wird eine möglichst konzentrierte methanolische Natriummethylatlösung mit einem Komplexbildner vorgelegt, und unter Abdestillieren des Methanols wird eine Lösung des Aldehyds der Formel III in einem hochsiedenden Lösungsmittel, wie insbesondere Dimethylformamid (DMF), Formylpiperidin oder Formylmorpholin, in Gegenwart eines Metallkatalysatorgemisches zugetropft.

Als Katalysatorgemisch werden vorzugsweise Kupfer(I)- und/oder Kupfer(II)-Salze mit einem ein- oder mehrzähnigen Liganden oder Gemische davon eingesetzt. Geeignete Liganden sind zum Beispiel Amine, wie insbesondere Morpholin, Aminoethylethanolamin, Tetraethylenpentamin, Trialkylphosphine, wie Triethyl- oder Trimethylphosphin, Ethylendiamintetraessigsäure (EDTA), und Aminosäuren, wie Lysin, Asparaginsäure oder N(2-Carboxyphenyl)glycin.

In der letzten Stufe des erfindungsgemäßen Verfahrens wird der in der zweiten Stufe erhaltene Syringaaldehyd vorzugsweise ohne dessen Isolierung nach Neutralisation des überschüssigen Alkalialkoholats mit einem anorganischen Säurechlorid, wie zum Beispiel Phosphoroxytrichlorid, in dem gleichen Reaktionsmedium mit einem üblichen Alkylierungsmittel alkyliert. Als solches wird vorzugsweise Dimethylsulfat oder 2-Methoxyethylchlorid verwendet. Die Alkylierung erfolgt vorzugsweise bei Temperaturen von 0 bis 60°C, insbesondere von 5 bis 30°C.

Nach einer weniger bevorzugten Ausführungsform des erfindungsgemäßen Verfahrens wird der Syringaaldehyd III als Natriumsalz isoliert und ohne Reinigung unter Phasentransferbedingungen mit einem der oben genannten Alkylierungsmittel alkyliert. Die Umsetzung bei Phasentransferbedingungen erfolgt nach allgemeinen Methoden, beschrieben in Synthesis 1973, Seiten 441 und 447.

Der in der letzten Stufe des erfindungsgemäßen Verfahrens erhaltene 3,5-Dimethoxy-4-alkoxybenzaldehyd wird nach Standardmethoden, beispielsweise Ausfällung und Umkristallisation, aus einem geeigneten Lösungsmittel, wie Isopropanol, isoliert.

Die Erfindung wird in den Beispielen erläutert.

## B e i s p i e l 1

a) Bromierung von Vanillin

Zu einem Gemisch aus 0,4 l Dichlorethan, 200 g Vanillin (1,31 mol), 2,6 g Succinimid, 0,2 l Wasser und 50 g Kaliumbromid gibt man bei maximal 40°C 120 g Brom innerhalb 1 h. Dann leitet man in 1 bis 3 h 44 g Chlor ein, bis ein Bromüberschuß sichtbar wird, der mit Sulfit zerstört wird. Man saugt bei 20°C ab und wäscht mit Wasser und Isopropanol nach.
Ausbeute: 266 g; Fp. 159,3 - 161,9°C

Aus der Dichlorethanmutterlauge läßt sich eine zweite Fraktion von 9,6 g gewinnen, so daß eine Ausbeute von 90,7% erreicht wird.

b) Herstellung von 3,4,5-Trimethoxybenzaldehyd

In einer Destillationsapparatur werden vorgelegt: 0,49 l 5,5 mol Natriummethylat, 10 ml DMF, 3 g Ethylendiamintetraessigsäure und 3 ml Trimethylphosphit. Bei 90 bis 95°C läßt man in 1 bis 2 h eine 60°C warme Lösung aus 250 ml Dimethylformamid, 231 g Bromvanillin, 8 g Kupfer(II)-acetat, 5,5 g Lysinhydrochlorid und 4 ml Morpholin zutropen, während Methanol abdestilliert. Dann kocht man noch 2 h unter Rückfluß. Das Lösungsmittel wird im Vakuum möglichst weit abgezogen, und der Rückstand wird mit Wasser bei 80°C auf 0,9 l aufgefüllt. Nach Zusatz von 50 g Kochsalz saugt man bei 20°C ab und wäscht mit 5% NaCl-Lösung nach. Das rohe Syringaaldehyd-Natriumsalz wird in 100 ml Wasser, 200 ml Toluol und 3 ml einer 25%igen Lösung von Cetyltrimethylammoniumchloridlösung suspendiert und bei 50°C mit 1 mol Dimethylsulfat versetzt. Nach 30 min gibt man die Hälfte einer Lösung aus 72 g Pottasche in 70 ml Wasser zu. Nach 15 min werden 0,5 mol DMS, der Rest der Pottaschelösung und wieder 0,5 mol DMS zugegeben, wobei $CO_2$ entweicht. Man rührt 1 h bei 50°C, bis kein $CO_2$ mehr entweicht, und zerstört überschüssiges Dimethylsulfat mit Ammoniak.

Die obere organische Phase wird abgetrennt, mit verd. HCl gewaschen und eingeengt. Den Sumpf kristallisiert man aus Isopropanol und saugt bei 5°C ab.
Ausbeute: 122 g; Fp. 73,9 - 75,8°C

Aus der Mutterlauge läßt sich eine zweite Fraktion von 28 g gewinnen. Gesamtausbeute: 150 g = 76,5% d.Th.

## B e i s p i e l 2

a) Bromierung von 4-Hydroxybenzaldehyd

Zu einem Gemisch aus 3 l Dichlorethan, 1,2 l Wasser, 240 g Kaliumbromid, 6 g Succinimid und 734 g p-Hydroxybenzaldehyd (6 mol) gibt man in 0,5 h 13,2 Val Brom (336 ml), wobei die Temperatur auf 60°C steigt. In 2 h leitet man 10,8 Val Chlor (383 g) bei 60 bis 80°C ein, kocht 1/2 h unter Rückfluß, zerstört den Bromüberschuß mit Sulfit und saugt bei 10°C ab. Nach Waschen mit Wasser und Isopropanol erhält man 1,47 kg 3,5-Dibrom-4-hydroxybenzaldehyd.
Fp. 183 - 183,4°C

Aus der Mutterlauge lassen sich noch 69 g gewinnen.
Ausbeute: 1,52 kg = 91% d.Th.

b) Herstellung von 3,4,5-Trimethoxybenzaldehyd

In 3,6 mol Natriummethylatlösung (656 ml), 20 ml DMF und 3 ml Phosphorigsäuretrimethylester werden 56 g 3,5-Dibrom-4-benzaldehyd bei 90°C vorgelegt. In 2 bis 3 h tropft man bei 90 - 95°C unter Destillatabnahme eine erwärmte Lösung aus 223 g 3,5-Dibrom-4-hydroxybenzaldehyd, 8 g Kupfer(II)acetat, 10 ml Morpholin und 14,8 g Ethylendiamintetraessigsäure in 0,4 l DMF so zu, daß das Gemisch gut rührfähig bleibt. Man läßt noch 2 h nachreagieren, kühlt auf 40 bis 60°C und neutralisiert mit 30 ml $POCl_3$. Dann zieht man etwa 0,2 l Lösungsmittel ab. Man kühlt auf 5°C, gibt 2 mol DMS hinzu. Bei 10

bis 20°C tropft man 0,6 mol Natriummethylatlösung in 1 h zu, wobei am Ende die Temperatur auf 20°C steigen soll. Dann gibt man bei 20 bis 30°C noch so viel Natriummethylatlösung zu, daß das Gemisch ständig alkalisch reagiert. Dann zieht man den Rest der Lösungsmittel möglichst weit ab, gibt 0,2 l Toluol zu und füllt mit Wasser auf 1 l auf. Die weitere Aufarbeitung erfolgt wie in Stufe b) von Beispiel 1.

Gesamtausbeute: 141 g = 72% d.Th.

## PATENTANSPRÜCHE

1. Verfahren zur Herstellung von 3,5-Dimethoxy-4-alkoxybenzaldehyden der allgemeinen Formel I

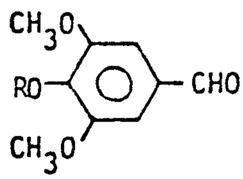

(I)

in der R eine Niedrigalkyl- oder Niedrigalkoxyethylengruppe mit maximal 6 Kohlenstoffatomen bedeutet, dadurch g e k e n n z e i c h n e t, daß man einen 4-Hydroxybenzaldehyd der allgemeinen Formel II

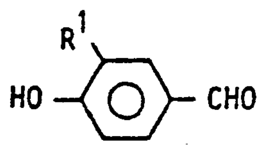

(II)

in der $R^1$ für ein Wasserstoffatom oder eine Methoxygruppe steht, zu einem Aldehyd der allgemeinen Formel III

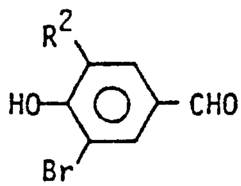

(III)

in der $R^2$ eine Methoxygruppe oder ein Bromatom bedeutet, bromiert, den erhaltenen Aldehyd der Formel II mit Alkalialkoholat in Gegenwart eines Metallkomplexes in Syringaaldehyd der Formel IV

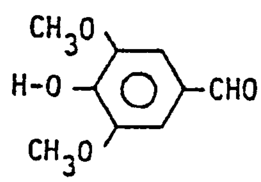

(IV)

überführt und daß man den erhaltenen Syringaaldehyd zu dem gewünschten 3,5-Dimethoxy-4-alkoxybenzaldehyd alkyliert.

2. Verfahren nach Anspruch 1, dadurch gekennzeichnet, daß man in der ersten Stufe die Bromierung des Aldehyds der Formel II in einem Gemisch eines chlorierten Kohlenwasserstoffs und Wasser in Gegenwart eines Alkalibromids und eines Halogenüberträgers, wie zum Beispiel eines Amids oder Imids, bei 10 bis 80°C durchführt.

3. Verfahren nach Anspruch 1 oder 2, dadurch gekennzeichnet, daß man in der zweiten Stufe beim Austausch der Bromatome im Aldehyd der Formel III Natriummethylat und eine katalytische Menge Trimethylphosphit vorlegt und bei 90 bis 95°C unter Destillatabnahme eine Lösung des Aldehyds der Formel III in einem dialkylierten Formamid in Gegenwart eines Kupferkomplexes eingibt.

4. Verfahren nach den Ansprüchen 1 bis 3, dadurch gekennzeichnet, daß man in der zweiten Stufe ein Kupfer(I)- oder Kupfer(II)salz mit Liganden, wie einem Amin, Phosphitderivaten, einer Aminosäure, bevorzugt Lysin oder EDTA oder Gemischen derselben, einsetzt.

5. Verfahren nach den Ansprüchen 1 bis 4, dadurch gekennzeichnet, daß man das Zwischenprodukt der Formel III ohne Isolierung nach Neutralisation des überschüssigen Natriummethylats mit einem anorganischen Säurehalogenid, wie zum Beispiel $POCl_3$, mit einem üblichen Alkylierungsmittel, wie zum Beispiel Dimethylsulfat oder 2-Methoxyethylchlorid, alkyliert.

6. Verfahren nach Anspruch 1, dadurch gekennzeichnet, daß man den Syringaaldehyd der Formel IV als Natriumsalz isoliert und ohne Reinigung unter Phasentransferbedingungen alkyliert.

0155335

Europäisches Patentamt

# EUROPÄISCHER RECHERCHENBERICHT

Nummer der Anmeldung

EP 84 10 3119

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | SYNTHESIS, 1983, Seite 308; D.V. RAO et al.: "An efficient synthesis of 3,4,5-trimethoxybenzaldehyde from Vanillin" * Seite 308 * | 1 | C 07 C 45/71<br>C 07 C 45/70<br>C 07 C 45/63<br>C 07 C 47/575<br>C 07 C 47/565 |
| D,Y | US-A-4 218 567 (P.S. MANCHAND et al.) * Beispiele 1,3 * | 1,2 | |
| A | CH-A- 572 879 (HOFFMANN-LA ROCHE) * Seiten 1-5 * | 1,2,5 | |
| D,A | DE-C- 723 412 (FAHLBERG-LIST AG CHEMISCHE FABRIKEN) * Seite 2 * | 1,3,4 | **RECHERCHIERTE SACHGEBIETE (Int. Cl.4)** |
| A | US-A-2 496 803 (D. McMILLAN) * Seiten 1,2 * | 1,6 | C 07 C 45/00<br>C 07 C 47/00 |

Der vorliegende Recherchenbericht wurde für alle Patentansprüche erstellt.

| Recherchenort | Abschlußdatum der Recherche | Prüfer |
|---|---|---|
| DEN HAAG | 12-11-1984 | BONNEVALLE E.I.H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument